Europäisches Patentamt

European Patent Office

Office européen des brevets

(19)

(11) Publication number: **0 661 280 A1**

## EUROPEAN PATENT APPLICATION

(12)

(21) Application number: **94120650.0**

(22) Date of filing: **24.12.94**

(51) Int. Cl.⁶: **C07D 295/12**, C07D 213/81,
C07D 213/82, C07D 307/68,
C07D 209/36, C07D 213/64,
C07D 311/46, C07C 311/19,
C07D 309/32, C12Q 1/44

(30) Priority: **29.12.93 JP 349879/93**

(43) Date of publication of application:
**05.07.95 Bulletin 95/27**

(84) Designated Contracting States:
**CH DE FR GB IT LI**

(71) Applicant: **KYOTO DAIICHI KAGAKU CO., LTD.**
**57 Nishiaketa-cho**
**Higashikujo**
**Minami-ku**
**Kyoto-shi**
**Kyoto-fu (JP)**

(72) Inventor: **Yagi, Yuji, c/o Kyoto Daiichi Kagaku**
**Co., Ltd.**
**57, Nishiaketa-cho,**
**Higashikujo,**
**Minami-ku**
**Kyoto-shi,**
**Kyoto-fu (JP)**

(74) Representative: **von Kreisler, Alek,**
**Dipl.-Chem. et al**
**Patentanwälte**
**von Kreisler-Selting-Werner**
**Bahnhofsvorplatz 1 (Deichmannhaus)**
**D-50667 Köln (DE)**

(54) Novel amino acid ester and reagent composition for detecting leucocytes or elastase in body liquid.

(57) An amino acid ester of the formula: (X-O-A-)ₙ-Y in which X is derived from an aromatic or heterocyclic compound: X-OH or its derivative; A is a residue of a L-amino acid, n is an integer of at least 2, and Y is a N-substituent of an amino acid derived from a compound having the same or different two or more carbonyl or sulfonyl groups which is formed from a single or complex compound selected from the group consisting of noncyclic or cyclic hydrocarbons having 1 to 10 carbon atoms, saturated or unsaturated heterocyclic compounds, aromatic compounds, organosilicon compounds having 1 to 10 silicon atoms, monosaccharides and oligosaccharide comprising 2 to 10 monosaccharides, all of which may have a substituent and/or a hetero atom to form the complex, which ester is specifically hydrolyzed by leucocytes or elastase.

EP 0 661 280 A1

BACKGROUND OF THE INVENTION

Field of the Invention

The present invention relates to a novel amino acid ester and a reagent composition comprising said ester for detecting leucocytes or elastase in a body liquid such as urine.

Description of the Related Art

In the diagnosis of diseases of kidneys and urinary and genital organs, detection and measurement of leucocytes in urine are very important. Hitherto, the detection of leucocytes in urine is carried out by counting the number of leucocytes in a specific volume of urine using a microscope. However, this counting method using the microscope is time-consuming and requires much labor. Further, unless the counting is carried out by a skilled person, it is impossible to count the number of leucocyles with high accuracy.

As an accurate and simple method instead of counting the number of leucocytes using the microscope, there is developed a method using an ester decomposition and/or protein decomposition activity of leucocytes. For example, in the method disclosed in Japanese Patent Publication No. 45982/1986, a substituted phenoxy amino acid ester is used as a substrate and a substituted phenol which is liberated by hydrolysis is detected with a diazonium salt. In the method disclosed in Japanese Patent Publication No. 41710/1987, a 5-phenylpyrrole amino acid ester is used as a substrate and 5-phenylpyrrole which is liberated by hydrolysis is detected with a diazonium salt. In the method disclosed in Japanese Patent Publication No. 43480/1990, an indoxyl amino acid ester used as a substrate and indoxyl which is liberated by hydrolysis is detected with a diazonium salt.

The methods disclosed in the above Japanese Patent Publications utilize the ester decomposition and/or protein decomposition activity of leucocytes. As a basic technology for these methods, a non-specific esterase dyeing method using an esterase substrate (e.g. naphthol ester, indoxyl ester, etc) and a chromophore (a diazonium salt) was established in the field of histochemistry or cytochemistry (cf. Pears, Histochemistry, Theoretical and Applied, 3rd Ed., Churchill Livingstone (1968) and F. Schmalzl and H. Braunsteinner, Klin. Wschr., 46, 642 (1968)).

However, since the urine contains various enzymes which have the ester decomposition and/or protein decomposition activity, it is apparent that specificity of the above methods is very low (cf. W. P. Raab, Clin. Chem., 18 (1), 5-25 (1972)).

SUMMARY OF THE INVENTION

One object of the present invention is to provide a novel amino acid ester which can be well decomposed by leucocytes or elastase for the specific detection of leucocytes or elastase in a body liquid but is not substantially hydrolyzed by other enzymes having the ester decomposition and/or protein decomposition activity present in the body fluid, in particular, urine, that is, an amino acid ester which is not hydrolyzed by the other enzymes or is hydrolyzed by the other enzymes at a lower rate than by leucocytes or elastase.

Another object of the present invention is to provide a reagent comprising said novel amino acid ester for detecting leucocytes or elastase in a body liquid such as urine.

According to a first aspect of the present invention, there is provided a novel amino acid ester of the formula:

$$(X-O-A-)_n-Y \qquad (I)$$

wherein X is derived from an aromatic or heterocyclic compound of the formula: X-OH or its derivative, which couples with a diazonium compound to develop a color; A is a residue of a L-amino acid, n is an integer of at least 2, and Y is a N-substituent of an amino acid derived from a compound having the same or different two or more carbonyl or sulfonyl groups, which is formed from a single or complex compound selected from the group consisting of noncyclic or cyclic hydrocarbons having 1 to 10 carbon atoms, saturated or unsaturated heterocyclic compounds, aromatic compounds, organosilicon compounds having 1 to 10 silicon atoms, monosaccharides and oligosaccharide comprising 2 to 10 monosaccharides, all of which may have a substituent and/or a hetero atom to form the complex.

According to a second aspect of the present invention, there is provided a reagent composition for detecting leucocytes or elastase in a body fluid, comprising the above novel amino acid ester and a diazonium salt.

When the amino acid ester (I) of the present invention is used together with the diazonium salt in the detection of leucocytes or elastase in the body fluid, it will react specifically with the leucocytes or elastase without the interference by any other enzyme having an ester decomposition and/or protein decomposition activity present in the body fluid. Thereby, the leucocytes and/or elastase can be detected or analyzed with high accuracy.

DETAILED DESCRIPTION OF THE INVENTION

In the formula (I), the group X is a group of the aromatic or heterocyclic compound represented by the formula: X-OH, which couples with a diazonium compound to develop a color.

Specific examples of the aromatic compound are phenol or its derivatives, 3-carbazolole, 2,3-di-(dimethylamino)phenol, 2,4-di(dimethylamino)phenol, 4-dimethylamino-2-methoxyphenol, 4-dimethylamino-2-methylphenol, 3-dimethylamino-2-methylphenol, 3-piperidinophenol, 3-dimethylamino-5-piperidinophenol, 3-methyl-5-piperidinophenol, 3-methoxy-5-piperidinophenol, 3-morpholinophenol, 3-dimethylamino-5-morpholinophenol, 3-methyl-5-morpholinophenol, 3-methoxy-5-morpholinophenol, 3-morpholino-5-piperidinophenol, 1,2,3,4-tetrahydroquinoxalin-5-ol, sesamol, 8-hydroxyjulolidine, ferulic acid, anthraflavic acid, ethyl-4-hydroxy-6-methyl-2-oxo-3-cyclohexane-1-carbohydrate, tropolon, 2-hydroxy-1,4-naphthoquinone, naphthol and its derivatives, naphthoresorcinol, and so on. Specific examples of the heterocyclic compound are 4-hydroxycumarine, 2-hydroxypyridine, 3-hydroxypyridine, 4-hydroxypyridine, 3-diazauridine, 3-dimethylamino-5-hydroxypyrazole, 5,6-dihydro-4-hydroxy-6-methyl-2H-pyran-2-on, 5-hydroxyuridine, kojic acid, mimosine, xanthopterin, 3-indoxyl, 3-hydroxy-5-phenylpyrrole, 3-methyl-5-hydroxyisoxazole, 5-dimethylaminomethyl-3-hydroxyisoxazole, 4,6-dihydroxypyrimidine 3-hydroxyisoquinoline, resorufin, and so on.

The group A is a residue of a L-amino acid. Examples of the L-amino acid are L-alanine, L-valine, L-leucine, L-glycine, and so on. Among them, L-alanine is preferred.

In the formula (I), n is an integer of at least 2, preferably 2, 3 or 4.

The group Y represents a N-substituent of the amino acid and is derived from a compound having the same or different two or more carbonyl groups or sulfonyl groups, which is formed from a single or complex compound selected from the group consisting of noncyclic or cyclic hydrocarbons having 1 to 10 carbon atoms, saturated or unsaturated heterocyclic compounds (e.g. tetrahydrofuran, pyrrolidine, piperazine, furan, thiophene, pyrrole, pyrazole, isoxazole, isothiazole, imidazole, oxazole, thiazole, pyridine, pyrazine, acridine, carbazole, quinoline, etc.), aromatic compounds (e.g. benzene, naphthalene, anthracene, etc.), organosilicon compounds having 1 to 10 silicon atoms, monosaccharides (e.g. D-glucose, D-galactose, D-glucosamine, etc.), and oligosaccharide comprising 2 to 10 monosaccharides (e.g. D-maltose, D-maltotriose, D-pentachitobiose, etc.). Any one of the above compounds may be substituted with one or more substituents such as a lower alkyl, alkenyl or alkoxy group having 1 to 10 carbon atoms, an aryl group having 6 to 14 carbon atoms, an amino group, an imino group, a hydroxyl group, or a halogen atom. To form the complex, the compound may have a hetero atom such as a nitrogen atom, an oxygen atom or a sulfur atom.

The amino acid ester of the present invention may be prepared by combining conventional methods as follows:

To obtain the compound of the formula (I), first a compound of the formula:

$$(HO\text{-}A\text{-})_n\text{-}Y \qquad (Ia)$$

$$(CH_3\text{-}O\text{-}A)_n\text{-}Y \qquad (Ib)$$

or

$$(CH_3CH_2\text{-}O\text{-}A)_n\text{-}Y \qquad (Ic)$$

wherein A is a residue of an amino acid, or a precursor of A is a free amino acid or a methyl or ethyl ester of an amino acid, Y is a N-substituent of an amino acid or a precursor of Y is a compound having the same or different two or more carbonyl or sulfonyl groups, and n are the same as defined above is synthesized. The compound (Ia) is a free amino acid, while the compounds (Ib) and (Ic) are the methyl and ethyl derivatives.

To synthesize the compound (Ia), (Ib) or (Ic), any one of well known processes for the synthesis of an acid amide or an acid imide may be used. For example, a carboxylic acid which is a precursor of the group Y and an amino acid which is a precursor of the group A are reacted through dehydration by heating or using a condensation reagent such as carbodiimide (Experimental Chemistry Lectures (Jikken Kagaku Koza), 4th Ed., Vol. 22, pages 138-144 and 259-262, edited by the Japan Chemical Society (Maruzen Kabushikikaisha, 1992), an acid which is a precursor of the group Y has been changed to an acid halide such as a carbonyl chloride or a sulfonyl chloride or an acid halide and is reacted with an amino acid (ibid, Vol. 22, pages 144-166), an acid which is a precursor of the group Y and has been changed to an acid anhydride or an acid anhydride is reacted with an amino acid (ibid, Vol. 22, pages 146-148), a carboxylic acid ester or lactone is used as a precursor of the group Y and reacted with an amino acid (ibid, Vol. 22, pages 148-150), an acid imide is synthesized using an acid anhydride as a precursor of the group Y (ibid, Vol. 22, pages 166-173), or a sulfonamide is synthesized from a sulfonyl-related compound as a precursor of the group Y (ibid, Vol. 24, pages 394-419). Further, the amino acid ester of the present invention may be synthesized using a conventional synthesis method of a protected amino acid (ibid, Vol. 22, pages 228-258).

Then, the ester linkage in the formula (I) between the carboxylic acid moiety of the group A and the aromatic compound or the heterocyclic group of the formula: X-OH is formed by a conventional esterification reaction such as a direct esterification by reacting $(HO-A-)_n$-Y and X-OH (ibid, Vol. 22, pages 43-45), a reaction between an acid anhydride or halide of $(HO-A-)_n$-Y and X-OH (ibid, Vol. 22, lines 50-51) or a transesterification of $(CH_3-O-A-)_n$-Y or $(CH_3CH_2-O-A-)_n$-Y with X-OH (ibid, Vol. 22, lines 52-53). Thereby the final product, namely the amino acid ester of the formula (I) is obtained.

Specific examples of the amino acid ester (I) of the present invention are as follows:

Butylene-1,4-di[N-carbonyl-L-alanyloxy-3-dimethylamino-5-piperidinophenol] (Compound 1)
Butylene-1,4-di[N-carbonyl-L-valyloxy-3-dimethylamino-5-piperidinophenol] (Compound 2)
Butylene-1,4-di[N-carbonyl-L-leucyloxy-3-dimethylamino-5-piperidinophenol] (Compound 3)
Butylene-1,4-di[N-carbonyl-L-glycyloxy-3-dimethylamino-5-piperidinophenol] (Compound 4)
Cyclohexene-1,4-di[N-carbonyl-L-alanyloxy-3-dimethylamino-5-piperidinophenol] (Compound 5)
1,2-Diphenylvinylene-1,2-di[N-carbonyl-L-alanyloxy-3-dimethylamino-5-piperidinophenol] (Compound 6)
Benzene-1,4-di[N-carbonyl-L-alanyloxy-3-dimethylamino-5-piperidinophenol] (Compound 7)
Benzene-1,4-di[N-oxycarbonyl-L-alanyloxy-3-dimethylamino-5-piperidinophenol] (Compound 8)
Naphthalene-2,6-di[Ncarbonyl-L-alanyloxy-3-dimethylamino-5-piperidinophenol] (Compound 9)
2,2'-Dithiobenzene-1,1'-di[N-carbonyl-L-alanyloxy-3-dimethylamino-5-piperidinophenol] (Compound 10)
Propane-1,2,3-tri[N-carbonyl-L-alanyloxy-3-dimethylamino-5-piperidinophenol] (Compound 11)
Benzene-1,2,4,5-tetra[N-carbonyl-L-alanyloxy-3-dimethylamino-5-piperidinophenol] (Compound 12)
Pyridine-2,6-di[N-carbonyl-L-alanyloxy-3-dimethylamino-5-piperidinophenol] (Compound 13)
6,6'-Dithiopyridine-3,3'-di[N-carbonyl-L-alanyloxy-3-dimethylamino-5-piperidinophenol] (Compound 14)
Benzene-1,2,4,5-di[N,N-dicarbonyl-L-alanyloxy-3-dimethylamino-5-piperidinophenol] (Compound 15)
Furan-3,4-di[N-carbonyl-L-alanyloxy-3-dimethylamino5-piperidinophenol] (Compound 16)
Benzene-1-[N-carbonyl-L-alanyloxy-3-dimethylamino-5-piperidinophenol]-4-[N-sulfonyl-L-alanyloxy-3-dimethylamino-5-piperidinophenol] (Compound 17)
Benzene-1,3-di(N-sulfonyl-L-alanyloxy-3-indole] (Compound 18)
Naphthalene-2,6-di-[N-sulfonyl-L-alanyloxy-3-indole] (Compound 19)
Diphenylmethane-4,4'-di[N-sulfonyl-L-alanyloxy-3-indole] (Compound 20)
Ethylene-1,2-di[N-(2-oxyethylene)sulfonyl-L-alanyloxy-3-indole] (Compound 21)
Tetra(ethylenedioxy)-O,O'-di[N-(4-benzenesulfonyl)-L-alanyloxy-3-indole] (Compound 22)
Naphthalene-1,3,6-tri[N-sulfonyl-L-alanyloxy-3-indole] (Compound 23)
Piperazine-1,4-di[N-ethylsulfonyl-L-alanyloxy-3-indole] (Compound 24)
Benzene-1,3-di[N-sulfonyl-L-alanyloxy-2-pyridine] (Compound 25)
Benzene-1,3-di[N-sulfonyl-L-alanyloxy-4-cumarin] (Compound 26)
Benzene-1,3-di[N-sulfonyl-L-alanyloxy-tropolon] (Compound 27)
Benzene-1,3-di[N-sulfonyl-L-alanyloxy-3-dimethylamino-5-pyrazole] (Compound 28)
Benzene-1,3-di[N-sulfonyl-L-alanyloxy-4-(5,6-dihydro-6-methyl-2H-pyran-2-on] (Compound 29)
Benzene-1,3-di[N-sulfonyl-L-alanyloxy-2-(1,4-naphthoquinone)] (Compound 30)

The reagent composition of the present invention comprises the amino acid ester of the present invention and a diazonium salt as essential components.

To prepare the reagent of the present invention, the amino acid ester and the diazonium salt are dissolved in a volatile solvent (e.g. water, methanol, acetone, toluene, dimethylformamide, etc.) together with one or more of conventionally used other components (e.g. a buffer, a wetting agent, a surfactant, etc.), and with a solution, a porous inert carrier (e.g. a filter paper, a glass fiber sheet, a synthetic non-woven fabric, a plastic foam, a fabric, a sintered metal, etc.) is impregnated and dried, for example, with a hot air drier, by

4

freeze drying or IR irradiation.

While it is possible to impregnate the porous carrier with all the components and dry it in one step, usually water-soluble components (e.g. the buffer, the wetting agent, the surfactant, etc.) are impregnated and dried in the first step and less water-soluble components such as the amino acid ester and the diazonium salt are impregnated and dried.

The carrier impregnated with the components can be used as such, while it is cut to a suitable size and fixed to a support with a double-coated tape, an adhesive or a hot melt resin or a physical method.

The preparation method of the reagent of the present invention is not limited to the impregnation method.

A concentration of the amino acid ester or the diazonium salt in an impregnation solution is from 0.1 to 10 mmol/l, preferably from 0.5 to 5 mmol/l. An amount of the buffer is selected so that pH in the carrier is from 6 to 11, preferably from 7 to 9 when the porous carrier is dipped in the body fluid.

Alternatively, the reagent of the present invention can be formed in a tablet form by mixing the amino acid ester and the diazonium salt together in a synthetic polymer (e.g. polyvinyl alcohol, polyvinylpyrrolidone, methyl vinyl ether-maleic anhydride copolymer, aminoalkyl methacrylate copolymer, hydroxypropylcellulose, carboxymethylcellulose, etc.) or a natural polymer (e.g. gelatin, alginic acid, cellulose, chitin, starch, pullulan, etc.) together with conventionally used additives (e.g. a buffer, a wetting agent, a surfactant, etc.), and molding the mixture by a tabletting machine to form a reagent tablet.

In addition, the reagent strip can be produced by dissolving or suspending the above mixture in a volatile solvent to prepare a homogeneous solution or a suspension, coating it on a plastic sheet or film and drying it.

When the body fluid is analyzed using the reagent of the present invention, the amino acid ester and the diazonium salt are preferably contained in the reagent composition in such amounts that each of them is present in the mixture of the reagent composition and the body fluid at a concentration of 0.1 to 10 mmol/l, more preferably 0.5 to 5 mmol/l. The buffer is preferably contained in the reagent composition in such an amount that pH of the mixture of the reagent composition and the body fluid is from 6 to 11, preferably from 7 to 9.

A kind of the diazonium salt is not limited. The diazonium salt should not react with bilirubin or urobilinogen and not develop any color and also not react with the amino acid ester (I) while it condensation reacts with a hydroxyl compound X-OH which is generated by the hydrolysis of the amino acid ester (I) to form a coloring material. Preferred examples of the diazonium salt are p-diazo-N,N-diethyl-m-toluidine, 1-diazo-2-naphthol-4-sulfonic acid, 5-diazomeldrum's acid, 2-methoxy-4-morpholinobenzenediazonium, Fast Black-BB, Fast Black-RR, Fast Red-ITR, Fast Red-KL, Fast Red-RC, Fast Red-TR, Fast Red-AL, Fast Red-PDC, Fast Corinth-V, Fast Garnet-GBC, Fast Black-K, Fast Violet-B, Fast Red Violet-LB, and so on.

PREFERRED EMBODIMENTS OF THE INVENTION

The present invention will be illustrated by the following Examples.

Example 1

Preparation of butylene-1,4-di[N-carbonyl-L-alanyloxy-3-dimethylamino-5-piperidinophenol] (Compound 1):

Butylene-1,4-di[N-carbonyl-L-alanine]

L-Alanine (2.00 g, 22.5 mmol) was dissolved in pyridine (30 ml) and cooled to 0 °C. To the solution, a solution of adipoyl dichloride [adipoyl dichloride (1.37 g, 7.5 mmol) in chloroform (20 ml)] was added portion by portion while stirring.

After keeping the mixture at 0 °C for 1 hour, it was stirred at room temperature for further 16 hours.

The reaction mixture was poured in chloroform (300 ml) and pH of the aqueous layer was adjusted to 1 to 2 with 1N hydrochloric acid while cooling with ice. After phase separation, the chloroform layer was washed with diluted hydrochloric acid five times and then with water five times. The chloroform layer was dried over anhydrous magnesium sulfate and filtrated followed by evaporation of the solvent under reduced pressure to obtain the above entitled compound (1.89 g, 6.56 mmol). Yield: 87.5 %.

H-NMR ($\delta$ppm, CDCl$_3$): 1.35 (d, J = 6.6 Hz, 6H), 1.50-1.90 (m, 4H), 2.10-2.60 (m, 4H), 4.15 (q, J = 6.6 Hz, 2H), 5.20-5.60 (m, 2H).

3-Dimethylamino-5-piperidinophenol

1,3,5-Trihydroxybenzene dihydrate (25.0 g, 154 mmol) was dissolved in dimethylformamide (269 ml). To the solution, water (116 ml) was added to obtain a homogeneous solution and then a 50 % aqueous solution of dimethylamine (17.6 g, 195 mmol) was added followed by stirring at room temperature for 48 hours.

The mixture was concentrated under reduced pressure. The residue (47.0 g) was isolated and purified by silica gel column chromatography to obtain 3-dimethylaminoresorcinol (17.5 g, 114 mmol). Yield: 74.2 %.

Thereafter, 3-dimethylaminoresorcinol (17.5 g) was dissolved in piperidine (175 ml) and reacted in an autoclave (180°C) for 12 hours.

Excessive piperidine was removed under reduced pressure. The residue (14.8 g) was isolated and purified by silica gel column chromatography three times to obtain the above entitled compound (4.0 g, 18.2 mmol). Yield: 16.0 %.

H-NMR ($\delta$ppm, CDCl$_3$): 1.40-1.80 (m, 6H), 2.85 (s, 6H), 2.9-3.2 (m, 4H), 5.70-6.00 (m, 4H).

Butylene-1,4-di[N-carbonyl-L-alanyloxy-3-dimethylamino-5-piperidinophenol]

Butylene-1,4-di[N-carbonyl-L-alanine] (1.50 g, 5.2 mmol) was suspended in thionyl chloride (20 ml) and heated for 90 minutes while stirring. Thionyl chloride was evaporated off under reduced pressure to obtain crude butylene-1,4-di[N-carbonyl-L-alanyl chloride] (1.69 g, 5.2 mmol). Yield of crude product: 100 %.

Crude butylene-1,4di[N-carbonyl-L-alanyl chloride] (1.69 g, 5.2 mmol) was dissolved in chloroform (30 ml).

Separately, 3-dimethylamino-5-piperidinophenol (3.44 g, 15.6 mmol) was dissolved in pyridine (50 ml). To this solution which was cooled to 0°C, the above solution of crude butylene-1,4-di[N-carbonyl-L-alanyl chloride] was added portion by portion while stirring.

After keeping the mixture at 0°C for 1 hour, it was stirred at room temperature for further 24 hours.

The reaction mixture was poured in chloroform (300 ml) and washed with diluted hydrochloric acid, an aqueous 5 % sodium hydrogencarbonate and water each five times.

The chloroform solution was dried over anhydrous magnesium sulfate and filtrated followed by evaporation of the solvent under reduced pressure to obtain the above entitled compound (2.37 g, 3.42 mmol). Yield: 65.8 %. Yield based on adipoyl dichloride: 57.6

H-NMR ($\delta$ppm, CDCl$_3$): 1.40-1.90 (m, 22H), 2.10-2.60 (m, 4H), 2.87 (s, 12H), 2.90-3.208 (m, 6H), 4.20 (m, 2H), 5.70-6.00 (m, 6H).

Example 2

Preparation of butylene-1,4-di[N-carbonyl-L-valyloxy-3-dimethylamino-5-piperidinophenol] (Compound 2):

In the same manner as in Example 1 except that L-valine was used in place of L-alanine, the entitled compound was prepared. Yielded amount: 2.62 g (3.50 mmol). Yield based on adipoyl dichloride: 59.3 %.

H-NMR ($\delta$ppm, CDCl$_3$): 1.40-1.90 (m, 16H), 2.00-2.70 (m, 4H), 2.88 (s, 12H), 2.90-3.20 (m, 8H), 3.97 (d, J = 4.8 Hz, 2H), 4.70-4.90 (m, 2H), 5.60-6.10 (m, 8H).

Example 3

Preparation of butylene-1,4-di[N-carbonyl-L-leucyloxy-3-dimethylamino-5-piperidinophenol] (Compound 3):

In the same manner as in Example 1 except that L-leucine was used in place of L-alanine, the entitled compound was prepared. Yielded amount: 2.55 g (3.28 mmol). Yield based on adipoyl dichloride: 54.2 %.

H-NMR ($\delta$ppm, CDCl$_3$): 1.08 (d, J = 6.0 Hz, 12H), 1.20-1.90 (m, 22H), 2.10-2.60 (m, 4H), 2.83 (s, 12H), 2.90-3.20 (m, 8H), 4.60-4.90 (m, 2H), 5.60-6.00 (m, 8H).

Example 4

Preparation of butylene-1,4-di[N-carbonyl-L-glycyloxy-3-dimethylamino-5-piperidinophenol] (Compound 4):

In the same manner as in Example 1 except that L-glycine was used in place of L-alanine, the entitled compound was prepared. Yielded amount: 1.95 g (2.94 mmol). Yield based on adipoyl dichloride: 56.5 %.

H-NMR ($\delta$ppm, CDCl$_3$): 1.40-1.90 (m, 16H), 2.10-2.60 (m, 4H), 2.88 (s, 12H), 2.90-3.20 (m, 8H), 3.75 (s, 4H), 5.60-6.10 (m, 8H).

## Example 5

Preparation of cyclohexene-1,4-di[N-carbonyl-L-alanyloxy-3-dimethylamino-5-piperidinophenol] (Compound 5):

### Cyclohexene-1,4-dicarbonyl chloride

Cyclohexene-1,4-dicarboxylic acid (1.29 g, 7.5 mmol) was suspended in thionyl chloride (20 ml) and heated for 90 minutes while stirring. After evaporating thionyl chloride off under reduced pressure, crude cyclohexene-1,4-dicarbonyl chloride (1.79 g, 7.5 mmol) was obtained. Yield of crude product: 100 %.

Thereafter, in the same manner as in Example 1 except that crude cyclohexene-1,4-dicarbonyl dichloride was used in place of adipoyl dichloride, the entitled compound was prepared. Yielded amount: 2.24 g (3.11 mmol). Yield based on cyclohexene-1,4-dicarboxylic acid: 50.4 %.

H-NMR ($\delta$ppm, CDCl$_3$): 1.10-2.60 (m, 20H), 2.84 (s, 12H), 2.90-3.50 (m, 10H), 4.00-4.30 (m, 2H), 5.50-6.00 (m, 8H).

## Example 6

Preparation of 1,2-diphenylvinylene-1,2-di[N-carbonyl-L-alanyloxy-3-dimethylamino-5-piperidinophenol] (Compound 6):

In the same manner as in Example 5 except that 1,2-diphenylvinylene-1,2-dicarboxylic anhydride was used in place of cyclohexene-1,4-dicarboxylic acid, the entitled compound was prepared. Yielded amount: 2.29 g (2.81 mmol). Yield based on 1,2-diphenylvinylene-1,2-dicarboxylic anhydride: 34.0 %.

H-NMR ($\delta$ppm, CDCl$_3$): 1.40-1.70 (m, 18H), 2.85 (s, 12H), 2.90-3.20 (m, 8H), 4.23 (m, 2H), 5.70-6.00 (m, 8H), 7.40-7.70 (m, 10H).

## Example 7

Preparation of benzene-1,4-di[N-carbonyl-L-alanyloxy-3-dimethylamino-5-piperidinophenol] (Compound 7):

In the same manner as in Example 1 except that terephthalyl dichloride was used in place of adipoyl dichloride, the entitled compound was prepared. Yielded amount: 2.00 g (2.80 mmol). Yield based on terephthalyl dichloride: 42.1 %.

H-NMR ($\delta$ppm, CDCl$_3$): 1.40-1.70 (m, 18H), 2.81 (s, 12H), 2.90-3.20 (m, 8H), 4.20 (m, 2H), 5.70-6.00 (m, 8H), 8.05 (s, 4H).

## Example 8

Preparation of benzene-1,4-di[N-oxycarbonyl-L-alanyl-oxy-3-dimethylamino-5-piperidinophenol] (Compound 8):

In the same manner as in Example 5 except that benzene-1,4-dioxycarboxylic acid was used in place of cyclohexene-1,4-dicarboxylic acid, the entitled compound was prepared. Yielded amount: 2.14 g (2.87 mmol). Yield based on benzene-1,4-dioxycarboxylic acid: 43.1 %.

H-NMR ($\delta$ppm, CDCl$_3$): 1.40-1.70 (m, 18H), 2.78 (s, 12H), 2.90-3.20 (m, 8H), 4.20 (m, 2H), 5.70-6.00 (m, 8H), 6.90-7.30 (s, 4H).

## Example 9

Preparation of naphthalene-2,6-di[N-carbonyl-L-alanyl-oxy-3-dimethylamino-5-piperidinophenol] (Compound 9):

In the same manner as in Example 5 except that naphthalene-2,6-carboxylic acid was used in place of cyclohexene-1,4-dicarboxylic acid, the entitled compound was prepared. Yielded amount: 2.27 g (2.98 mmol). Yield based on naphthalene-2,6-dicarboxylic acid: 37.2 %.

H-NMR ($\delta$ppm, CDCl$_3$): 1.40-1.70 (m, 18H), 2.87 (s, 12H), 2.90-3.20 (m, 8H), 4.31 (m, 2H), 5.70-6.00 (m, 8H), 7.65-8.10 (m, 4H), 8.65 (s, 2H).

Example 10

Preparation of 2,2'-dithiobenzene-1,1'-di[N-carbonyl-L-alanyloxy-3-dimethylamino-5-piperidinophenol] (Compound 10):

In the same manner as in Example 5 except that 2,2'-dithiodibenzoic acid was used in place of cyclohexene-1,4-dicarboxylic acid, the entitled compound was prepared. Yielded amount: 2.40 g (2.81 mmol). Yield based on 2,2'-dithiodibenzoic acid: 31.7 %.

H-NMR ($\delta$ppm, CDCl$_3$): 1.40-1.70 (m, 18H), 2.88 (s, 12H), 2.90-3.20 (m, 8H), 4.33 (m, 2H), 5.70-6.00 (m, 8H), 7.00-8.20 (m, 8H).

Example 11

Preparation of propane-1,2,3-tri[N-carbonyl-L-alanyl-oxy-3-dimethylamino-5-piperidinophenol] (Compound 11):

In the same manner as in Example 5 except that propane-1,2,3-tricarboxylic acid was used in place of cyclohexene-1,4-dicarboxylic acid and crude propane-1,2,3-tricarbonyl dichloride and propane-1,2,3-tri[N-carbonyl-L-alanine] were charged in amounts of 5.0 mmol and 3.47 mmol, respectively, the entitled compound was prepared. Yielded amount: 1.34 g (1.35 mmol). Yield based on propane-1,2,3-tricarboxylic acid: 17.4 %.

H-NMR ($\delta$ppm, CDCl$_3$): 1.40-1.70 (m, 27H), 2.70-3.30 (m, 1H), 2.40-2.70 (m, 4H), 2.82 (s, 18H), 2.90-3.20 (m, 12H), 4.18 (m, 3H), 5.70-6.10 (m, 12H).

Example 12

Preparation of benzene-1,2,4,5-tetra[N-carbonyl-L-alanyloxy-3-dimethylamino-5-piperidinophenol] (Compound 12):

In the same manner as in Example 5 except that benzene-1,2,4,5-tetracarboxylic acid was used in place of cyclohexene-1,4-dicarboxylic acid and crude benzene-1,2,4,5-tetracarbonyl chloride and benzene-1,2,4,5-tetra[N-carbonyl-L-alanine] were charged in amounts of 3.75 mmol and 2.6 mmol, respectively, the entitled compound was prepared. Yielded amount: 1.25 g (0.93 mmol). Yield based on benzene-1,2,4,5-tetracarboxylic acid: 17.5 %.

H-NMR ($\delta$ppm, CDCl$_3$): 1.40-1.70 (m, 36H), 2.93 (s, 24H), 2.90-3.20 (m, 16H), 4.38 (m, 4H), 5.70-6.20 (m, 16H), 8.00 (s, 2H).

Example 13

Preparation of pyridine-2,6-di[N-carbonyl-L-alanyloxy-3-dimethylamino-5-piperidinophenol] (Compound 13):

In the same manner as in Example 5 except that pyridine-2,6-dicarbonyl chloride was used in place of crude cyclohexene-1,4-dicarboxylic acid, the entitled compound was prepared. Yielded amount: 2.52 g (3.53 mmol). Yield based on pyridine-2,6-dicarbonyl chloride: 47.3 %.

H-NMR ($\delta$ppm, CDCl$_3$): 1.40-1.70 (m, 18H), 2.77 (s, 12H), 2.90-3.20 (m, 8H), 4.13 (m, 2H), 5.65-6.05 (m, 8H), 8.38 (s, 3H).

Example 14

Preparation of 6,6'-dithiopyridine-3,3'-di[N-carbonyl-L-alanyloxy-3-dimethylamino-5-piperidinophenol] (Compound 14):

In the same manner as in Example 5 except that 6,6'-dithiodinicotinic acid was used in place of cyclohexene-1,4-dicarboxylic acid, the entitled compound was prepared. Yielded amount: 1.92 g (2.25 mmol). Yield based on 6,6'-dithiodinicotinic acid: 20.6 %.

H-NMR ($\delta$ppm, CDCl$_3$): 1.40-1.75 (m, 18H), 2.75 (s, 12H), 2.80-3.20 (m, 8H), 4.15 (m, 2H), 5.70-6.00 (m, 8H), 7.60-8.40 (m, 4H), 8.95 (s, 2H).

### Example 15

Preparation of benzene-1,2,4,5-di[N,N-dicarbonyl-L-alanyloxy-3-dimethylamino-5-piperidinophenol] (Compound 15):

#### Benzene-1,2,4,5-di[N,N-dicarbonyl-L-alanine]

To toluene (30 ml), 1,2,4,5-benzene tetracarboxylic anhydride (2.18 g, 10 mmol) and L-alanine (1.78 g, 20 mmol) were added and vigorously stirred under reflux for 30 minutes.

The reaction mixture was washed with a 5 % aqueous solution of sodium hydrogencarbonate, diluted hydrochloric acid and water each 5 times, dried over anhydrous magnesium sulfate, filtrated and concentrated under reduced pressure. The residue was isolated and purified by silica gel column chromatography to obtain the entitled compound (3.08 g, 8.54 mmol). Yield: 85.4 %.

H-NMR ($\delta$ppm, CDCl$_3$): 1.38 (d, J = 6.6 Hz, 6H), 4.21 (q, J = 6.6 Hz, 2H), 8.05 (s, 2H).

Thereafter, in the same manner as in Example 1 except that benzene-1,2,4,5-di[N,N-dicarbonyl-L-alanine] was used in place of butylene-1,4-di[N-carbonyl-L-alanine], the entitled compound was prepared. Yielded amount: 2.65 g (3.46 mmol). Yield based on 1,2,4,5-benzene tetracarboxylic acid: 56.9 %.

H-NMR ($\delta$ppm, CDCl$_3$): 1.45-1.75 (m, 18H), 2.82 (s, 12H), 2.95-3.25 (m, 8H), 4.38 (m, 2H), 5.90-6.20 (m, 6H), 8.10 (s, 2H).

### Example 16

Preparation of furan-3,4-di[N-carbonyl-L-alanyloxy-3-dimethylamino-5-piperidinophenol] (Compound 16):

In the same manner as in Example 5 except that furan-3,4-dicarboxylic acid was used in place of cyclohexene-1,4-dicarboxylic acid, the entitled compound was prepared. Yielded amount: 2.29 g (3.26 mmol). Yield based on furan-3,4-dicarboxylic acid: 46.1 %.

H-NMR ($\delta$ppm, CDCl$_3$): 1.35-1.65 (m, 18H), 2.74 (s, 12H), 2.80-3.10 (m, 8H), 4.13 (m, 2H), 5.60-5.90 (m, 8H), 8.51 (s, 2H).

### Example 17

Preparation of benzene-1-[N-carbonyl-L-alanyloxy-3-dimethylamino-5-piperidinophenol]-4-[N-sulfonyl-L-alanyloxy-3-dimethylamino-5-piperidinophenol] (Compound 17):

#### Benzene-1-[N-carbonyl-L-alanine]-4-[N-sulfonyl-L-alanine]

4-Sulfobenzoic acid (4.04 g, 20 mmol) was suspended in nitrobenzene (100 ml), and to the suspension, phosphorus penta-chloride (8.32 g, 40 mmol) was carefully added portion by portion. The mixture was gradually heated while stirring and stirred under reflux for 5 hours. After cooling to room temperature, the mixture was poured in a large amount of iced water while vigorously stirring. After stirring the mixture for 30 minutes, the nitrobenzene layer was separated.

Separately, L-alanine (5.34 g, 60 mmol) was dissolved in a 2 mol/l aqueous solution of sodium hydroxide (100 ml) which was ice cooled. To this solution, the above nitrobenzene layer was slowly dropwise added at 0 to 5°C while vigorously stirring.

After stirring at 0 to 5°C for 24 hours, the nitrobenzene layer was separated and washed with a 5 % aqueous solution of sodium hydrogencarbonate, diluted hydrochloric acid, again a 5 % aqueous solution of sodium hydrogencarbonate and water each 5 times, dried over anhydrous magnesium sulfate, filtrated and concentrated under reduced pressure. The residue was isolated and purified by silica gel column chromatography twice to obtain the entitled compound (1.80 g, 5.22 mmol). Yield: 26.1 %.

H-NMR ($\delta$ppm, CDCl$_3$): 1.34 (s, 3H), 1.41 (s, 3H), 4.00-4.30 (m, 2H), 5.40-6.10 (m, 2H), 8.12 (d, J = 3.0 Hz, 4H).

#### Benzene-1-[N-carbonyl-L-alanyloxy-3-dimethylamino-5-piperidinophenol]-4-[N-sulfonyl-L-alanyloxy-3-dimethylamino-5-piperidinonhenol]

Thereafter, in the same manner as in Example 1 except that benzene-1-[N-carbonyl-L-alanine]-4-[N-sulfonyl-L-alanine] was used in place of butylene-1,4-di[N-carbonyl-L-alanine], the entitled compound was prepared. Yielded amount: 2.50 g (3.34 mmol). Yield based on 4-sulfobenzoic acid: 16.8 %.

H-NMR ($\delta$ppm, CDCl$_3$): 1.37 (s, 3H), 1.45 (s, 3H), 4.05-4.35 (m, 2H), 5.45-6.15 (m, 2H), 8.06 (d, J = 3.2 Hz, 4H).

Example 18

Preparation of benzene-1,3-di[N-sulfonyl-L-alanyloxy-3-indole] (Compound 18):

Benzene-1,3-di[N-sulfonyl-L-alanine]

L-Alanine (2.00 g, 22.5 mmol) was dissolved in an aqueous solution of sodium hydroxide (1.50 g of sodium hydroxide in 20 ml of water), while benzene-1,3-disulfonyl chloride (2.06 g, 7.5 mmol) was dissolved in toluene (20 ml).

To the former solution which was ice cooled to keep the internal temperature at 15°C or lower with stirring, the latter solution was gradually added dropwise. The reaction mixture was stirred for 3 hours with keeping the temperature at 15°C or lower, during which pH of the reaction mixture was kept at 9 by the addition of a 1 mol/l aqueous solution of sodium hydroxide.

With 1N hydrochloric acid, pH of the aqueous layer was adjusted to 1 to 2 while cooling with ice. The precipitated material was recovered by filtration and washed with diluted hydrochloric acid and then water each 5 times. The material was dried under reduced pressure to obtain the entitled compound (2.06 g, 5.41 mmol). Yield: 72.1 %.

H-NMR ($\delta$ppm, CDCl$_3$): 1.52 (d, J = 7.2 Hz, 6H), 4.30-4.50 (m, 2H), 5.30-5.70 (m, 2H), 7.95-8.35 (m, 4H).

Benzene-1,3-di[N-sulfonyl-L-alanyloxy-3-indole]

In the same manner as in Example 1 except that benzene-1,3-di[N-sulfonyl-L-alanine] was used in place of butylene-1,4-di[N-carbonyl-L-alanine], crude benzene-1,3-di[N-sulfonyl-L-alanyloxy chloride] (2.17 g) was prepared. Yield of crude product: 100 %.

Indoxyl acetate (4.00 g, 22.8 mmol) was dissolved in methanol (85 ml). To this solution, an aqueous solution of sodium hydroxide (3.65 g (91.3 mmol) of sodium hydroxide in 46 ml of water) was added in a nitrogen atmosphere and stirred at room temperature for 30 minutes. Then, to the solution, an aqueous solution of citric acid (9.72 g of citric acid in 34 ml of water) was added. After evaporating methanol off under reduced pressure, the precipitated yellowish green crystal of 3-hydroxyindole was recovered by filtration and dried.

3-Hydroxyindole (1.52 g, 11.44 mmol) was dissolved in tetrahydrofuran (24 ml) and pyridine (11.2 ml) in a nitrogen atmosphere. To this solution, trifluoroacetic acid (1.56 ml) and anhydrous magnesium sulfate (630 mg) were added while cooling with ice. To the 3-hydroxyindole solution, a solution of crude benzene-1,3-di[N-sulfonyl-L-alanyloxy chloride] (2.17 g, 5.2 mmol) in tetrahydrofuran (16 ml) was added at 0°C, followed by stirring at 0°C for 1 hour and then at room temperature for 16 hours.

The reaction mixture was poured in ethyl acetate (300 ml) and washed with a 0.2 mol/l aqueous solution of citric acid four times and water once. After drying over anhydrous magnesium sulfate, the mixture was filtrated and the filtrate was concentrated under reduced pressure. The residue was isolated and purified by silica gel chromatography three times to obtain the entitled compound (0.79 g, 1.29 mmol). Yield: 24. 8 %. Yield based on benzene-1,3-disulfonyl chloride: 17.9 %.

H-NMR ($\delta$ppm, CDCl$_3$): 1.43 (d, J = 7.2 Hz, 6H), 4.00-4.40 (m, 2H), 5.40-6.00 (m, 2H), 6.70-7.40 (m, 10H), 7.95-8.35 (m, 6H).

Example 19

Preparation of naphthalene-2,6-di[N-sulfonyl-L-alanyl-oxy-3-indole] (Compound 19):

In the same manner as in Example 18 except that naphthalene-2,6-disulfonyl chloride was used in place of benzene-1,3-disulfonyl chloride, the entitled compound was prepared. Yielded amount: 0.57 g (0.87 mmol). Yield based on naphthalene-2,6-disulfonyl chloride: 16.8 %.

H-NMR ($\delta$ppm, CDCl$_3$): 1.47 (d, J = 7.2 Hz, 6H), 4.00-4.40 (m, 2H), 5.40-6.00 (m, 2H), 6.70-7.40 (m, 10H), 7.80 (s, 2H), 8.13 (s, 4H), 7.90-8.30 (m, 2H).

10

Example 20

Preparation of diphenylmethane-4,4'-di[N-sulfonyl-L-alanyloxy-3-indole] (Compound 20):

In the same manner as in Example 18 except that diphenyl-methane-4,4'-disulfonyl chloride was used in place of benzene-1,3-disulfonyl chloride, the entitled compound was prepared. Yielded amount: 0.60 g (0.86 mmol). Yield based on naphthalene-4,4'-disulfonyl chloride: 16.6 %.

H-NMR ($\delta$ppm, CDCl$_3$): 1.55 (d, J = 7.1 Hz, 6H), 3.70-3.90 (m, 4H), 4.35 (q, J = 7.1 Hz, 2H), 6.90-7.55 (m, 12H), 7.30 (d, J = 10 Hz, 4H), 7.76 (d, J = 10 Hz, 4H).

Example 21

Preparation of ethylene-1,2-di[N-(2-oxyethylene)-sulfonyl-L-alanyloxy-3-indole] (Compound 21):

In the same manner as in Example 18 except that ethylene-1,2-di(2-oxyethane)sulfonyl chloride was used in place of benzene-1,3-disulfonyl chloride, the entitled compound was prepared. Yielded amount: 0.60 g (0.93 mmol). Yield based on ethylene-1,2-di(2-oxyethane)sulfonyl chloride: 17.8 %.

H-NMR ($\delta$ppm, CDCl$_3$): 1.52 (d, J = 2.4 Hz, 6H), 3.20-3.40 (m, 4H), 3.45 (s, 4H), 3.60-3.90 (m, 4H), 4.40-4.50 (m, 2H), 5.80 (d, J = 2.4 Hz, 2H), 7.00-7.30 (m, 8H), 7.46 (d, J = 2.4 Hz, 2H), 8.35 (s, 2H).

Example 22

Preparation of tetra(ethylenedioxy)-O,O'-di[N-(4-benzene-sulfonyl)-L-alanyloxy-3-indole] (Compound 22):

In the same manner as in Example 18 except that tetra(ethylenedioxy)-O,O'-di[N-(4-benzenesulfonyl) chloride] was used in place of benzene-1,3-disulfonyl chloride, the entitled compound was prepared. Yielded amount: 0.78 g (0.90 mmol). Yield based on naphthalene-2,6-disulfonyl chloride: 17.4 %.

H-NMR ($\delta$ppm, CDCl$_3$): 1.46 (d, J = 7.2 Hz, 6H), 3.60 (s, 16H), 4.00-4.40 (m, 2H), 5.67 (d, J = 9.6 Hz, 2H), 6.65 (d, J = 9.8 Hz, 4H), 7.62 (d, J = 9.8 Hz, 4H), 6.70-7.40 (m, 10H), 8.65 (d, J = 3.0 Hz, 2H).

Example 23

Preparation of naphthalene-1,3,6-tri[N-sulfonyl-L-alanyl-oxy-3-indole] (Compound 23):

In the same manner as in Example 18 except that 1,3,6-naphthalenetrisulfonyl chloride was used in place of benzene-1,3-disulfonyl chloride, and crude naphthalene-1,3,6-trisulfonyl chloride and naphthalene-1,3,6-tri[N-sulfonyl-L-alanine] were charged in amounts of 5.0 mmol and 3.47 mmol, respectively, the entitled compound was prepared. Yielded amount: 0.59 g (0.64 mmol). Yield based on crude naphthalene-1,3,6-trisulfonyl chloride: 18.5 %.

H-NMR ($\delta$ppm, CDCl$_3$): 1.48 (d, J = 7.2 Hz, 9H), 4.05-4.45 (m, 3H), 5.40-6.00 (m, 3H), 6.70-7.40 (m, 15H), 7.70-8.20 (m, 5H), 8.40-8.65 (m, 3H).

Example 24

Preparation of piperazine-1,4-di[N-ethylsulfonyl-L-alanyloxy-3-indole] (Compound 24):

In the same manner as in Example 18 except that piperazine-1,4-diethylenesulfonyl chloride was used in place of benzene-1,3-disulfonyl chloride, the entitled compound was prepared. Yielded amount: 0.47 g (0.69 mmol). Yield based on piperazine-1,4-diethylenesulfonyl chloride: 13.5 %.

H-NMR ($\delta$ppm, CDCl$_3$): 1.43 (d, J = 7.2 Hz, 6H), 2.50-3.30 (m, 16H), 3.95-4.35 (m, 2H), 5.40-6.00 (m, 2H), 6.70-7.40 (m, 10H), 8.55-8.75 (m, 2H).

Example 25

Preparation of benzene-1,3-di[N-sulfonyl-L-alanyloxy-2-pyridine] (Compound 25):

In chloroform (30 ml), benzene-1,3-di[N-sulfonyl-L-alanyloxy chloride] (see Example 18) (2.17 g, 5.2 mmol) was dissolved. Separately, in pyridine (30 ml), 2-hydroxypyridine (1.48 g, 15.6 mmol) was dissolved and ice cooled. To the latter solution which was being stirred, the former solution was dropwise added with keeping the temperature at 10°C or lower, and they were reacted at 10°C or lower for 1 hour, followed by stirring at room temperature for 24 hours.

The reaction mixture was poured in chloroform (300 ml) and washed with diluted hydrochloric acid, a 5 % aqueous solution of sodium hydrogencarbonate and water each 5 times.

The mixture was then dried over anhydrous magnesium sulfate, filtrated and concentrated under reduced pressure. The residue was isolated and purified by silica gel chromatography twice to obtain the entitled compound (1.58 g, 3.42 mmol). Yield: 65.8 %. Yield based on benzene-1.3-disulfonyl chloride: 47.4 %.

H-NMR ($\delta$ppm, CDCl$_3$): 1.58 (s, 6H), 4.25-4.55 (m, 2H), 5.60-6.00 (m, 2H), 6.20-6.80 (m, 4H), 7.40-7.60 (m, 4H), 7.95-8.35 (m, 4H).

Example 26

Preparation of benzene-1,3-di[N-sulfonyl-L-alanyloxy-4-cumarin] (Compound 26):

In the same manner as in Example 25 except that 4-hydroxycumarin was used in place of 2-hydroxypyridine, the entitled compound was prepared. Yielded amount: 5.73 g (3.10 mmol). Yield based on benzene-1,3-disulfonyl chloride: 59.6 %.

H-NMR ($\delta$ppm, CDCl$_3$): 1.52 (s, 6H), 4.30-4.60 (m, 2H), 5.60-6.00 (m, 2H), 5.73 (s, 2H), 7.10-8.35 (m, 12H).

Example 27

Preparation of benzene-1,3-di[N-sulfonyl-L-alanyloxy-tropolone] (Compound 27):

In the same manner as in Example 25 except that tropolon was used in place of 2-hydroxypyridine, the entitled compound was prepared. Yielded amount: 1.81 g (3.50 mmol). Yield based on benzene-1,3-disulfonyl chloride: 67.3 %.

H-NMR ($\delta$ppm, CDCl$_3$): 1.48 (s, 6H), 4.25-4.55 (m, 2H), 5.50-6.00 (m, 2H), 6.80-7.50 (m, 10H), 7.90-8.35 (m, 4H).

Example 28

Preparation of benzene-1,3-di[N-sulfonyl-L-alanyloxy-3-dimethylamino-5-pyrazole] (Compound 28):

In the same manner as in Example 25 except that 3-dimethylamino-5-hydroxypyrazole was used in place of 2-hydroxy-pyridine, the entitled compound was prepared. Yielded amount: 1.28 g (2.43 mmol). Yield based on benzene-1,3-disulfonyl chloride: 46.7 %.

H-NMR ($\delta$ppm, CDCl$_3$): 1.48 (s, 6H), 2.65 (s, 12H), 4.25-4.55 (m, 2H), 5.40-6.00 (m, 2H), 7.60 (s, 2H), 7.90-8.35 (m, 4H).

Example 29

Preparation of benzene-1,3-di[N-sulfonyl-L-alanyloxy-4-(5,6-dihydro-6-methyl-2H-pyran-2-on] (Compound 29):

In the same manner as in Example 25 except that 5,6-dihydro-4-hydroxy-6-methyl-2H-pyran-2-on was used in place of 2-hydroxypyridine, the entitled compound was prepared. Yielded amount: 1.84 g (3.49 mmol). Yield based on benzene-1,3-disulfonyl chloride: 67.2 %.

H-NMR ($\delta$ppm, CDCl$_3$): 1.15 (d, J = 5.4 Hz, 4H), 1.44 (s, 6H), 2.20-2.50 (m, 4H), 3.10-3.70 (m, 2H), 4.25-4.55 (m, 2H), 5.40-6.00 (m, 2H), 5.78 (s, 2H), 7.90-8.35 (m, 4H).

Example 30

Preparation of benzene-1,3-di[N-sulfonyl-L-alanyloxy-2-(1,4-naphthoquinone)] (Compound 30):

In the same manner as in Example 25 except that 2-hydroxy-1,4-naphthoquinone was used in place of 2-hydroxypyridine, the entitled compound was prepared. Yielded amount: 2.07 g (3.34 mmol). Yield based on benzene-1,3-disulfonyl chloride: 64.3 %.

H-NMR ($\delta$ppm, CDCl$_3$): 1.48 (s, 6H), 5.60-5.80 (m, 2H), 7.80-8.55 (m, 14H).

Example 31

Preparation of a test strip for detecting leucocytes and elastase:

1) Preparation of a test paper

A 0.4 mol/l aqueous solution of boric acid and a 0.4 mol/l aqueous solution of borax were mixed to adjust pH to 8. To this buffer solution (50 ml), polyvinylpyrrolidone (K-30) (0.5 g) and sodium laurylsulfate (0.05 g) were dissolved. With this mixture, a filter paper (No. 514 A manufactured by Toyo Filter Paper Co., Ltd., 10 cm x 10 cm) was impregnated and dried in a circulation type fan drier at 80°C for 30 minutes to obtain Test Paper I.

In methanol (50 ml), n-decanol (1.2 g), 1-diazo-2-naphthol-4-sulfonicacid (12.5 mg, 0.05 mmol) and an amino acid ester (0.05 mmol) were dissolved. With this solution, Test Strip I was impregnated and dried in the circulation type fan drier at 50°C for 15 minutes to obtain Test Pater II.

Obtained Test Paper II was cut to a size of 5 x 5 mm and adhered to one end of a white polyethylene terephthalate strip (thickness of 100 μm, 5 x 70 mm) with a double-coated adhesive tape to obtain a test strip.

As the amino acid ester, each of Compounds 1 to 4 prepared in Examples 1-4, respectively was used.

2) Samples

Each of the following enzymes was dissolved in 0.1 mol/l Tris hydrochloric acid buffer (0.01 mol/l of calcium chloride, pH of 7.8) at a concentration of 10 mg/l to obtain each enzyme solution:

α-chymotrypsin (manufactured by Sigma, bovine pancreas), trypsin (manufactured by Sigma, bovine pancreas), cathepsin (manufactured by Sigma, bovine pancreas), subtilisin (manufactured by Boehringer Mannheim, Bacillus subtilis), elastase (manufactured by Sigma, human leucocyte), and butylcholinesterase ( (manufactured by Sigma, human serum).

From human whole blood, leucocytes (neutrophils) were isolated, purified and suspended in physiological saline at a concentration of 200 leucocytes/μl to obtain a leucocyte liquid.

3) Measurement

The test strip was dipped in each sample liquid and immediately removed. The excessive sample liquid was absorbed by a sheet of filter paper, and the test strip was kept in a temperature controlled vessel at 37°C to effect the reaction for 2 minutes. As a control, a Tris-buffer solution was used as a sample liquid.

A color developed on each test trip was observed by eyes.

When the Tris-buffer solution was used, no color change was observed on the test strip during the reaction.

4) Results

The results of the color observation by eyes are shown in Table 1.

From these results, it is apparent that the amino acid ester of the present invention is specifically hydrolyzed by the leucocyte or elastase.

EP 0 661 280 A1

Table 1

| Sample | Amino Acid Ester | | | |
| --- | --- | --- | --- | --- |
| | Compound 1 | Compound 2 | Compound 3 | Compound 4 |
| Leucocyte liquid | Red purple | Very slight red purple | Slight red purple | Slight red purple |
| $\alpha$-Chymotrypsin | No change | No change | No change | Slight red purple |
| Trypsin | No change | No change | No change | No change |
| Cathepsin | No change | No change | No change | No change |
| Subtilisin | Slight red purple | Very slight red purple | Very slight red purple | Slight red purple |
| Elastase | Red purple | Slight red purple | Very slight red purple | Red purple |
| Butylcholinesterase | No change | No change | No change | No change |

Example 32

Test paper for detecting leucocytes and elastase in urine

1) Preparation of test paper

A test paper was prepared in the same manner as in Example 31 except that the following items were changed:
Filter paper: 3 MMCHR manufactured by Whatman
Diazonium salt: 2-methoxy-4-morpholinobenzene diazonium salt (16.2 mg, 0.05 mmol)
Animo acid ester: Compound 18, 19, 20, 21 or 22 (prepared in each of Examples 18 to 22)

2) Preparation of comparative test paper

As a comparative amino acid ester to be compared with the amino acid esters of the present invention, N-tosilalanyloxy-3-indole (disclosed in Japanese Patent Publication No. 43480/1990) was used to prepare a test paper. This ester was prepared by the method of Example 6 of Japanese Patent Publication No. 3479/1984.
Except using this amino acid ester (0.05 mmol = 18 mg), a comparative test paper was prepared in the same manner as above 1).

3) Samples

The same samples as those used in Example 31 were used.
In addition, as urine samples, a normal urine (leucocyte negative) and pyuria (about 20 leucocytes/hpf in the urinarysediment method) were used.

4) Measurement

On the test strip, a drop of each sample (about 8 $\mu$l) was pipetted and reacted in the temperature controlled vessel at 37°C for 1 minute. Then, reflectances (R %) at two wavelengths of 560 and 750 nm were measured using a differential colorimeter (SZ-$\Sigma$90 manufactured by Nippon Denshoku Kogyo Kabushikikaisha).

14

5) Results

The results are shown in Table 2.

Table 2

| Sample | Amino acid ester (Compound No.) | | | | | |
|---|---|---|---|---|---|---|
| | 18 | 19 | 20 | 21 | 22 | Comparative |
| Leucocyte liquid | 72.6 | 77.4 | 51.9 | 52.6 | 50.4 | 39.4 |
| Pyuria | 73.3 | 79.0 | 50.8 | 51.0 | 49.5 | 37.5 |
| Normal urine | 93.6 | 96.7 | 87.5 | 87.8 | 88.1 | 86.6 |
| $\alpha$-Chymotripsin | 97.0 | 97.0 | 88.6 | 84.6 | 95.2 | 90.8 |
| Trypsin | 96.1 | 96.5 | 89.7 | 89.7 | 98.5 | 92.7 |
| Cathepsin | 95.3 | 97.7 | 90.3 | 92.8 | 98.9 | 97.0 |
| Subtilisin | 91.0 | 98.2 | 88.2 | 59.5 | 90.3 | 56.7 |
| Elastase | 67.2 | 74.5 | 34.9 | 33.8 | 38.1 | 31.1 |
| Butylcolinesterase | 90.4 | 98.3 | 89.8 | 94.7 | 97.9 | 97.0 |
| Tris-buffer | 95.5 | 98.2 | 90.9 | 95.5 | 98.6 | 99.6 |

From the results of Table 2, it is seen that the differences of reflectance ΔR % = R % of the sample - R % of the blank (the blank being the normal urine in the case of the urine samples, or the Tris-buffer in the case of the enzyme solutions) indicate that the test strips using the amino acid esters of the present invention were well color developed by the leucocyte and elastase.

Though the comparative test strip was color developed by elastase and leucocytes, it was also color developed by the solutions of $\alpha$-chymotrypsin, trypsin and subtilisin.

Accordingly, it is apparent that the amino acid esters of the present invention have the higher specificity to elastase and leucocytes than the comparative amino acid ester.

Example 33

Test for detecting leucocytes and elastase in urine

1) Preparation of test strip

In acetone (500 ml), n-decanol (10 g) was dissolved and then filter paper powder A (Toyo Filter Paper Co., Ltd.) (4.0 mg) was added. After being kept standing at room temperature for 10 minutes, the mixture was filtrated and a cake was spread over a vat and dried in the circulation type fan drier at 40°C for 20 minutes. After drying, the dried cake was charged in a polyethylene bag and comminuted (Powder I).

2-Methoxy-4-morpholinobenzene diazonium salt (4.9 g), boric acid (254 mg), borax (296 mg), sodium laurylsulfate (20 mg) and anhydrous silicic acid (800 mg) were ground and mixed in a mortar. The mixture was charged in a polyethylene bag, and Powder II was added and well mixed. The ground powder was pre-pelletized by a pelletizer (200 mg of the sample. Diameter of 13 mm. Pressure of 5 tons). The pellet was ground, mixed in the mortar and sieved to the particle sizes of 50 to 200 $\mu$m (Powder II).

In necessary, the amino acid ester was also ground in the mortar and sieved to the particle sizes of 50 to 200 $\mu$m.

Powder II and the amino acid ester (0.015 mmol) were charged in a polyethylene bag and mixed well, and the mixture was pelletized by the tabletting machine to obtain a tablet (100 mg of the sample. Diameter of 13 mm. Pressure of 1 ton).

As the amino acid ester, each of Compounds 11 to 15 prepared in Examples 11 to 15, respectively was used.

2) Samples

The same samples as those used in Example 32 were used.

3) Measurement

On the tablet, an amount of each sample (50 μl) was dropped and reacted in the temperature controlled vessel at 37°C for 2 minutes. Then, reflectances (R %) at two wavelengths of 540 and 750 nm were measured using the differential colorimeter (SZ-Σ90 manufactured by Nippon Denshoku Kogyo Kabushikikaisha).

4) Results

The results are shown in Table 3.

Table 3

| Sample | Amino acid ester (Compound No.) | | | | | |
|---|---|---|---|---|---|---|
| | 11 | 12 | 13 | 14 | 15 | Comparative |
| Leucocyte liquid | 88.0 | 89.7 | 78.5 | 82.2 | 86.9 | 31.5 |
| Pyuria | 86.7 | 88.4 | 76.6 | 81.0 | 85.5 | 32.8 |
| Normal urine | 91.9 | 93.5 | 93.2 | 92.0 | 94.2 | 85.7 |
| α-Chymotripsin | 96.7 | 95.0 | 89.6 | 90.4 | 92.2 | 89.7 |
| Trypsin | 96.4 | 95.9 | 93.5 | 93.7 | 93.4 | 91.4 |
| Cathepsin | 96.0 | 96.4 | 95.1 | 96.9 | 97.2 | 95.8 |
| Subtilisin | 89.8 | 90.0 | 80.4 | 87.9 | 88.1 | 37.4 |
| Elastase | 87.1 | 87.1 | 75.3 | 81.3 | 84.2 | 30.3 |
| Butylcolinesterase | 97.5 | 96.1 | 96.7 | 96.3 | 95.9 | 96.5 |
| Tris-buffer | 97.6 | 95.6 | 95.8 | 96.4 | 95.9 | 96.6 |

As in Example 32, from the results of Table 3, the differences of reflectance ΔR % = R % of the sample - R % of the blank (the blank being the normal urine in the case of the urine samples, or the Tris-buffer in the case of the enzyme solutions) indicate that the test strips using the amino acid esters of the present invention were well color developed by the leucocyte and elastase.

From the results in this Example, it is understood that the tablet form test strip of the present invention can also detect elastase and leucocytes.

Example 34

Preparation of a test strip for detecting leucocytes and elastase:

1) Preparation of a test strip

A 0.1 mol/l aqueous solution of boric acid containing 0.1 mol/l of potassium chloride (50 ml) and a 0.1 mol/l aqueous solution of sodium hydroxide (3.9 ml) were mixed. To the mixture, water was added to adjust the whole volume to 100 ml.

To this buffer solution (10 ml), polyvinyl alcohol (molecular weight of 13000 to 23000) (1.5 g) and polyethylene glycol (molecular weight of 4000) (0.5 g) were dissolved to obtain Solution I.

On a sand blasted white polybutylene terephthalate film (thickness of 50 μm), Solution I was coated at a thickness of 200 μm and dried in the circulation type fan drier at 60°C for 30 minutes to obtain Film I.

In methanol (10 ml), Fast Red-RC (27.3 ml, 0.1 mmol) and the amino acid ester (0.1 mmol) were dissolved to obtain Solution II. Separately, in methanol (10 ml), n-decanol (50 mg) was dissolved and then polyvinylpyrrolidone (K-90) (1.5 g) and polyethylene glycol (molecular weight of 4000) (0.5 g) were dissolved to obtain Solution III.

Solutions II and III were mixed and coated on Film I at a thickness of 200 $\mu$m and dried in the circulation type fan drier at 50°C for 20 minutes to obtain Film II.

Obtained Film II was cut to a size of 5 x 5 mm and adhered to one end of a white polyethylene terephthalate strip (thickness of 100 $\mu$m, 5 x 70 mm) with a double-coated adhesive tape to obtain a test strip.

As the amino acid ester, each of Compounds 26, 27, 28 and 30 prepared in Examples 26, 27, 28 and 30, respectively was used.

2) Samples

The same samples as those used in Example 31 were used.

3) Measurement

On the test strip, a drop of each sample (5 $\mu$l) was pipetted and reacted in the temperature controlled vessel at 37°C for 2 minutes. Then, the developed color was observed by the eyes by using the test strip added with the Tris-buffer as a reference. When the Tris-buffer was added, no color was developed.

4) Results

The results of the color observation by eyes are shown in Table 4.

From these results, it is apparent that the amino acid ester of the present invention is well color developed specifically by the leucocyte or elastase.

From the results in this Example, it is understood that this type of test strip of the present invention can also detect elastase and leucocytes.

Table 4

| Sample | Amino Acid Ester | | | |
|---|---|---|---|---|
| | Compound 26 | Compound 27 | Compound 29 | Compound 30 |
| Leucocyte liquid | Yellow | Reddish orange | Yellow | Reddish orange |
| α-Chymotrypsin | No change | No change | No change | No change |
| Trypsin | No change | No change | No change | No change |
| Cathepsin | No change | No change | No change | No change |
| Subtilisin | No change | No change | No change | No change |
| Elastase | Yellow | Reddish orange | No change | Reddish orange |
| Butylcholinesterase | No change | No change | No change | No change |

**Claims**

1. A amino acid ester of the formula:

(X-O-A-)$_n$-Y    (I)

wherein X is derived from an aromatic or heterocyclic compound of the formula: X-OH or its derivative; A is a residue of a L-amino acid, n is an integer of at least 2, and Y is a N-substituent of an amino acid derived from a compound having the same or different two or more carbonyl or sulfonyl groups which is formed from a single or complex compound selected from the group consisting of noncyclic or cyclic hydrocarbons having 1 to 10 carbon atoms, saturated or unsaturated heterocyclic compounds, aromatic compounds, organosilicon compounds having 1 to 10 silicon atoms, monosaccharides and oligosaccharide comprising 2 to 10 monosaccharides, all of which may have a substituent and/or a hetero atom to form the complex.

2. The amino acid ester according to claim 1, wherein A in the formula (I) is L-alanine.

3. The amino acid ester according to claim 1, wherein n in the formula (I) is 2.

4. The amino acid ester according to claim 1, wherein A in the formula (I) is L-alanine and n in the formula (I) is 2.

5. A reagent composition for detecting leucocytes or elastase in a body fluid, comprising an amino acid ester of the formula:

$(X-O-A-)_n-Y$     (I)

wherein X is derived from an aromatic or heterocyclic compound of the formula: X-OH or its derivative; A is a residue of a L-amino acid, n is an integer of at least 2, and Y is a N-substituent of an amino acid derived from a compound having the same or different two or more carbonyl or sulfonyl groups which is formed from a single or complex compound selected from the group consisting of noncyclic or cyclic hydrocarbons having 1 to 10 carbon atoms, saturated or unsaturated heterocyclic compounds, aromatic compounds, organosilicon compounds having 1 to 10 silicon atoms, monosaccharides and oligosaccharide comprising 2 to 10 monosaccharides, all of which may have a substituent and/or a hetero atom to form the complex, and a diazonium salt.

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | EP-A-0 158 204 (MILES LABORATORIES,INC) *Document* | 1 | C07D295/12 C07D213/81 C07D213/82 C07D307/68 C07D209/36 C07D213/64 C07D311/46 C07C311/19 C07D309/32 C12Q1/44 |
| A | EP-A-0 157 360 (MILES LABORATORIES,INC.) *Document* | 1 | |
| A | EP-A-0 157 362 (MILES LABORATORIES,INC.) *Document* | 1 | |
| A | EP-A-0 157 361 (MILES LABORATORIES,INC.) *Document* | 1 | |
| A | EP-A-0 039 880 (BOEHRINGER MANNHEIM GMBH) *Document* | 1 | |
| A | JP-A-5 168 497 (WAKO PURE CHEM IND LTD) *PAJ abstract* | 1 | |
| A | JP-A-1 139 564 (TERUMO CORP) *PAJ abstract* | 1 | |
| D,A | JP-A-61 045 982 (BOEHRINGER MANNHEIM GMBH) *Derwent abstract* | 1 | TECHNICAL FIELDS SEARCHED (Int.Cl.6) |
| D,A | JP-A-62 041 710 (MILES LABORATORIES) *Derwent abstract* | 1 | C07D C07C C12Q |
| D,A | JP-A-2 043 480 (BOEHRINGER MANNHEIM GMBH) *Derwent abstract* | 1 | |

The present search report has been drawn up for all claims

| Place of search | Date of completion of the search | Examiner |
|---|---|---|
| THE HAGUE | 13 March 1995 | Luyten, H |